(19) Europäisches Patentamt
European Patent Office
Office européen des brevets

(11) Numéro de publication: **0 173 585**
**B1**

# (12) FASCICULE DE BREVET EUROPEEN

(45) Date de publication du fascicule du brevet: **24.08.88**

(51) Int. Cl.⁴: **C 07 D 211/18**, C 07 D 401/06, A 61 K 31/445

(21) Numéro de dépôt: **85400438.9**

(22) Date de dépôt: **07.03.85**

(54) **Médicaments à base de dérivés de la pipéridine, nouveaux dérivés de la pipéridine et leurs procédés de préparation.**

(30) Priorité: **09.03.84 FR 8403670**

(43) Date de publication de la demande: **05.03.86 Bulletin 86/10**

(45) Mention de la délivrance du brevet: **24.08.88 Bulletin 88/34**

(84) Etats contractants désignés: **AT BE CH DE FR GB IT LI LU NL SE**

(56) Documents cités:
**EP - A - 0 012 643**

**JOURNAL OF THE CHEMICAL SOCIETY, partie III, 1954, pages 95-99; R.J. GILLESPIE et al.: "Solutions in sulphuric acid. Part XIV. Cryscopic measurements on some unsaturated aromatic ketones and aldehydes"**

**Le dossier contient des informations techniques présentées postérieurement au dépôt de la demande et ne figurant pas dans le présent fascicule.**

(73) Titulaire: **RHONE-POULENC SANTE, Les Miroirs 18 Avenue d'Alsace, F-92400 Courbevoie Cédex (FR)**

(72) Inventeur: **Renault, Christian, 61 rue des Mallets, F-95150 Taverny (FR)**
Inventeur: **Mestre, Michel, 1 rue Scheffer, F-75116 Paris (FR)**

(74) Mandataire: **Gaumont, Robert et al, RHONE-POULENC RECHERCHES Service Brevets Pharma 25, Quai Paul Doumer, F-92408 Courbevoie Cedex (FR)**

**EP 0 173 585 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention a pour objet des médicaments utiles pour le traitement et/ou la prévention des troubles du rythme qui contiennent en tant que principe actif un composé de formule générale (I) dans laquelle X représente un groupe -CO-, -CHOH- ou -CH-$NH_2$ et Ar représente un reste aromatique choisi parmi les cycles A, B, C ou D dans lesquels R représente l'atome d'hydrogène ou un groupe alkyle comportant 1 à 4 atomes de carbone, Y et Z, identiques ou différents représentent chacun l'atome d'hydrogène ou un groupe alcoxy comportant 1 à 3 atomes de carbone ainsi que leurs sels d'addition avec un acide minéral ou organique pharmaceutiquement acceptable, les nouveaux dérivés de formule (I) et leurs procédés de préparation.

Des dérivés de la phényl-1-(pipéridyl-4)-3-propanone-1 utilisés notamment comme antiarythmiques sont décrits dans le brevet européen 12643.

Les composés de formule (I) pour lesquels X représente le groupe -CO- et Ar représente le reste napthyl-1 ou isoquinolyl-1 ont déjà été décrits (N.V. Rubtsov, Zhur, obshchei, khim, 1953, 23, 1893 et G.R. Clemo, J. Chem. Soc. 1954, 95) cependant, aucune propriété pharmacologique ou application thérapeutique n'a été signalée pour ces produits. Les composés de formule (I) autres que ceux cités précédemment sont nouveaux et font partie en tant que tels de l'invention.

Ce sont les composés de formule (I) dans laquelle soit X représente le groupe -CHOH- ou -CH-$NH_2$ et Ar représente un reste aromatique choisi parmi les 4 cycles A, B, C et D mentionnés précédemment soit X représente le groupe -CO- et Ar représente un reste aromatique choisi parmi les cycles A, C et D mentionnés précédemment excepté que Y et Z ne peuvent pas représenter tous les deux un atome d'hydrogène ou les sels de ces composés avec des acides minéraux ou organiques.

Comme exemples de sels ont peut citer, les chlorhydrates, sulfates, nitrates, phosphates, acétates, propionates, succinates, benzoates, fumarates, maléates, méthanesulfonates, salicylates.

Les composés de formule (I) pour lesquels X représente le groupe -CO- peuvent être préparés par condensation, en présence d'une base d'un ester de formule (II) avec un ester d'acide (pipéridinyl-4) propionique de formule (III) puis hydrolyse et décarboxylation du composé de formule (IV) ainsi obtenu.

Dans les formules (II), (III) et (IV) Ar représente un reste aromatique choisi parmi les cycles A, B, C et D mentionnés précédemment, $R_1$ et $R_2$ représentent des groupes alkyle de bas poids moléculaire par exemple méthyle ou éthyle et B représente un groupe protecteur de la fonction amine, stable en milieu alcalin anhydre et susceptible d'être éliminé en milieu acide comme ceux décrits par R.A. Boissonnas, Advances in Organic Chemistry 3, p. 159, Interscience (1963); on utilise avantageusement le groupe benzoyle ou benzyloxycarbonyle.

La réaction de condensation est effectuée selon des procédés décrits dans «The acetoacetic acid ester condensation» C.R. Hauser et col., Organic Reactions, vol. 1, p. 226 Wiley and Sons, 1942.

On opère avantageusement en présence d'une base telle qu'un alcoolate comme le tertiobutylate de potassium ou un hydrure métallique comme l'hydrure de sodium ou de potassium, au sein d'un solvant inerte tel qu'un hydrocarbure ou un solvant aprotique comme le tétrahydrofuranne, à une température variant de 0 °C à la température d'ébullition du solvant utilisé.

La réaction d'hydrolyse est conduite selon les procédés décrits dans «Cleavage of β-keto-esters», R.B. Wagner et H.D. Zook, Synthetic Organic Chemistry, p. 327, Wiley and Sons, 1953.

La méthode la plus avantageuse consiste à chauffer à l'ébullition le produit de formule (IV) dans une solution aqueuse d'un acide tel que l'acide chlorhydrique ou l'acide sulfurique.

Les composés de formule (I) dans laquelle X représente le groupe -CHOH- peuvent être préparés par réduction des composés correspondants de formule (I) dans laquelle X représente le groupe -CO-.

Une méthode de réduction consiste à utiliser comme agent de réduction un hydrure métallique réducteur tel que ceux mentionnés dans «Complex hydrides and related reducing agents in organic synthesis» A. Hajos, Elsevier Scientific Publishing Company, Amsterdam, Oxford, New York 1979.

Parmi les agents réducteurs on peut citer les borohydrures de métaux alcalins tels que le borohydrure de sodium ou de potassium qui sont utilisés à la température ambiante au sein d'un solvant tel qu'un alcool comme le méthanol ou l'éthanol, un mélange eau-alcool ou le tétrahydrofuranne ou l'hydrure d'aluminium et de lithium qui est utilisé au sein d'un solvant inerte tel que l'éther, le tétrahydrofuranne ou un hydrocarbure à une température variant de 0 °C à la température d'ébullition du solvant.

Les composés de formule (I) pour lesquels X représente le groupe -CH-$NH_2$ peuvent être préparés à partir des composés de formule (I) pour lesquels X représente le groupe -CO- par les méthodes décrites par C.A. Buehler, et D.E. Pearson, Survey of organic Synthesis, vol. 1, p. 427, Wiley Interscience 1970.

Un procédé particulièrement avantageux consiste à traiter le composé cétonique par le formiate d'ammonium à une température variant de 150 à 200 °C puis hydrolyser en milieu acide.

Le mélange réactionnel obtenu dans les procédés énumérés ci-dessus, est traité suivant des méthodes classiques, physiques (évaporation, extraction, distillation, cristallisation, chromatographie...) ou chimiques (formation de sels et régénération de la base...) afin d'isoler les composés de formule (I) à l'état pur soit sous forme de la base libre soit sous forme d'un sel de celle-ci avec un acide minéral ou organique.

Les exemples suivants illustrent la préparation des composés de formule (I).

Exemple 1: (naphthyl-2)-1-(piperidyl-4)-3-propanone-1

A 5,5 g de naphtoate-2 d'éthyle et 10 ml d'une suspension à 20% d'hydrure de potassium dans l'huile, dans 25 ml de tétrahydrofuranne anhydre à l'ébullition on ajoute, sous azote, une solution de 7 g de (benzoyl-1-pipéridine-4)-3-propionate d'éthyle dans 25 ml de tétrahydrofuranne anhydre.

Après 20 heures d'ébullition et refroidissement on ajoute 5 ml d'éthanol et élimine les solvants sous pression réduite.

On reprend le résidu avec 50 ml d'eau, 50 ml d'acide chlorhydrique 11N et 50 ml d'acide acétique et porte l'ensemble à l'ébullition pendant 8 heures. Après refroidissement on dilue à l'eau, lave la phase aqueuse à l'éther, l'alcalinise au moyen d'une solution 12N d'hydroxyde de sodium et extrait 2 fois avec 200 ml d'éther.

On lave la phase éthérée à l'eau, la sèche sur du sulfate de magnésium anhydre et l'évapore à sec sous pression réduite. On obtient 2,7 g de produit brut que l'on reprend dans 20 ml d'éthanol auxquels on ajoute de l'éther chlorhydrique.

Après filtration, lavage du précipité et séchage on obtient 2,5 g de (naphthyl-2)-1-(pipéridyl-4)-3-propanone-1 sous forme de chlorhydrate fondant à 225°C.

Exemple 2: (naphthyl-1)-1-(piperidyl-4)-3-propanone-1

Sous azote on introduit 8 ml d'une suspension d'hydrure de potassium à 50% dans l'huile et 25 ml de tétrahydrofuranne anhydre. On ajoute 3 g de naphtoate-1 d'éthyle et porte le mélange au reflux. On ajoute ensuite une solution de 2,9 g de (benzoyl-1-pipéridine-4)-3 propionate d'éthyle en solution dans 25 ml de tétrahydrofuranne. On laisse 5 heures au reflux, refroidit, ajoute un peu d'éthanol et évapore le milieu réactionnel à sec. On reprend le résidu dans 50 ml d'acide chlorhydrique 6N et 25 ml d'éthanol et porte à nouveau au reflux pendant 36 heures. On extrait le milieu réactionnel à l'éther, l'alcalinise au moyen d'une solution d'hydroxyde de sodium 12N et l'extrait à l'acétate d'éthyle. On lave la phase organique à l'eau, la sèche sur du sulfate de magnésium et l'évapore à sec sous pression réduite. On obtient 1,54 g de produit sous forme d'huile qu'on transformera en chlorhydrate au sein de l'acétone. On obtient la (naphthyl-1)-1-(pipéridyl-4)-3-propanone-1 sous forme de chlorhydrate fondant à 171°C.

Exemple 3: (dimethoxy-6,7-isoquinolyl-1)-1-(piperidyl-4)-3-propanone-1

A 14 g d'hydrure de sodium dans 200 ml de tétrahydrofuranne anhydre, sous azote, on ajoute une solution de 40,8 g de diméthoxy-6,7-isoquinoléinecarboxylate-1 d'éthyle et de 37,7 g de (benzoyl-1-pipéridine-4)-3-propionate d'éthyle dans 250 ml de tétrahydrofuranne anhydre. On porte le mélange 4 heures au reflux puis après refroidissement à 0 °C l'hydrolyse avec 90 ml d'acide chlorhydrique 6N. On évapore le tétrahydrofuranne sous pression réduite, ajoute 200 ml d'acide chlorhydrique 6N et porte de nouveau au reflux 18 heures. On lave la phase aqueuse avec 2 fois 200 ml d'éther, l'alcalinise avec 150 ml d'ammoniaque concentré et l'extrait plusieurs fois au chloroforme. On lave la phase chloroformique à l'eau, la sèche sur du sulfate de magnésium et l'évapore à sec sous pression réduite. On obtient 45 g de produit que l'on chromatographie sur du gel de silice en utilisant un mélange chloroforme-diéthylamine (9:1) comme éluant. On obtient 30 g de produit que l'on dissout dans 200 ml d'éthanol. On amène la solution à pH 1 avec une solution d'éthanol chlorhydrique. On ajoute 200 ml d'acétone ce qui entraine la cristallisation du produit. Après filtration, lavage des cristaux à l'acétone et à l'éther et séchage on obtient 15 g de (diméthoxy-6,7-isoquinolyl-1)-1-(pipéridyl-4)-3-propanone-1 sous forme de chlorhydrate fondant à 228°C.

Le diméthoxy-6,7 isoquinoléinecarboxylate-1 d'étzhyle peut être préparé selon T. KAMETANI et coll., Chem. Abst. 1967, 66, 28632 u.

Exemple 4: [(dimethyl-1,1 ethyl)-2quinazolinyl-4]-1 (piperidyl-4)-3 propanone-1

Sous azote on place 6,5 g d'hydrure de sodium à 80% dans l'huile avec 60 ml de tétrahydrofuranne anhydre.

On agite et ajoute une solution de 22,4 g de (diméthyl-1,1-éthyl)-2-quinazolinecarboxylate-4 d'éthyle dans 150 ml de tétrahydrofuranne anhydre. On agite 30 min et ajoute une solution de 12,6 g de (benzoyl-1-pipéridine-4)-3-propionate d'éthyle dans 100 ml de tétrahydrofuranne anhydre. On agit 20 heures à la température ambiante, ajoute 50 ml d'éthanol et évapore le solvant sous pression réduite. On reprend le résidu à l'eau et extrait la phase aqueuse 4 fois à l'acétate d'éthyle. On sèche la phase organique sur du sulfate de magnésium anhydre et l'évapore à sec, on obtient 29 g de produit que l'on reprend dans 100 ml d'éthanol et 100 ml d'acide chlorhydrique 6N.

Après 50 heures d'ébullition, on évapore l'éthanol et reprend le résidu par du chlorure de méthylène et de l'eau. On alcalinise la phase aqueuse au moyen d'une solution d'hydroxyde de sodium 12N.

L'huile qui relargue est extraite par du chlorure de méthylène, et l'extrait organique est séché sur du sulfate de magnésium puis évaporé à sec sous pression réduite. On obtient 17,3 g de produit que l'on chromatographie sur du gel de silice en utilisant un mélange chloroforme-diéthylamine (95:5) comme éluant.

On recueille 8,8 g de [(diméthyl-1,1-éthyl)-2-quinazolinyl-4]-1-(pipéridyl-4)-3-propanone-1 sous forme d'huile dont le spectre de RMN du proton dans $CDCl_3$ présente les caractéristiques suivantes:

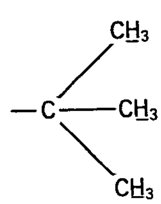
δ = 1,5 ppm
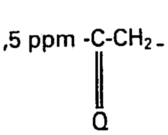
δ = 3,3 ppm

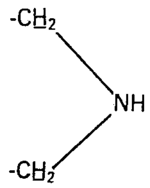
δ = 2,6 et 3,1 ppm

$H_5$ (quinazolinyl) δ = 8,1 ppm
$H_8$ (quinazolinyl) δ = 8,7 ppm
$H_6$ et $H_7$ (quinazolinyl) δ = 7,7 et 7,9 ppm
Le reste des protons est compris entre 0,9 et 1,9 ppm.

Un échantillon recristallisé dans l'éther éthylique fond à 127 °C.

Le (diméthyl-1,1-éthyl)-2-quinazolinecarboxylate-4 d'éthyle peut être préparé comme suit:

A 10 g d'aniline et 16,4 g de triéthylamine dans 80 ml de chloroforme on ajoute, en refroidissant 19,5 g de chlorure de pivaloyle.

Après 3 heures d'agitation on ajoute de l'eau et on amène le pH à 10 au moyen d'hydroxyde de sodium. On décante et extrait la phase aqueuse au chloroforme. Les phases organiques rassemblées sont lavées à l'eau, séchées sur du sulfate de magnésium et évaporées à sec sous pression réduite. Le résidu est repris dans l'éther, on filtre le solide et le sèche. On obtient 14,5 g de produit auquel on ajoute 13 ml de chlorure de thionyle. Le mélange est porté à 90 °C pendant 3 heures. On élimine l'excès de chlorure de thionyle par distillation et ajoute 10 ml de cyanoformiate d'éthyle et 11,8 ml de chlorure stannique. On porte l'ensemble 10 mn à 130 °C. Après refroidissement on dissout le résidu dans du chlorure de méthylène, lave la phase organique à l'eau, la sèche sur du sulfate de magnésium et l'évapore à sec sous pression réduite. On reprend le résidu par 200 ml d'éther isopropylique, filtre un insoluble et évapore le filtrat. On obtient 18,8 g d'une laque que l'on chromatographie sur du gel de silice en utilisant un mélange cyclohexane-acétate d'éthyle (9:1) comme éluant.

On recueille 17,5 g de (diméthyl-1,1-éthyl)-2-quinazolinecarboxylate-4 d'éthyle fondant à 46–47 °C.

Exemple 5: (naphthyl-1)-1-(pipéridyl-4)-3-propanol-1

A 12 g de (naphthyl-1)-1-(pipéridyl-4)-3-propanone-1 dans 200 ml de méthanol on ajoute, en 30 min, 2 g de borohydrure de sodium.

Après 1 heure d'agitation on ajoute de l'acide chlorhydrique 5N jusqu'à pH 3, concentre à sec sous pression réduite, redissout le résidu dans l'eau et lave la phase aqueuse à l'acétate d'éthyle. On alcalinise la phase aqueuse et l'extrait avec 3 fois 250 ml de chloroforme. On lave l'extrait organique à l'eau, le sèche sur du sulfate de magnésium et l'évapore à sec sous pression réduite. On obtient 11,5 g de produit que l'on chromatographie sur du gel de silice au moyen d'un mélange chloroforme-diéthylamine (9:1) comme éluant. On receuille 7,5 g de produit que l'on dissout dans de l'acétone. On traite par de l'éther chlorhydrique et on obtient ainsi 5,1 g de (naphthyl-1)-1-(pipéridyl-4)-3-propanol-1 sous forme de chlorhydrate fondant à 165 °C.

Exemple 6: (isoquinolyl-1)-1-(pipéridyl-4)-3-propanol-1

A 11,8 g d'(isoquinolyl-1)-1-(pipéridyl-4)-3-propanone-1 dans 120 ml d'éthanol on ajoute 1,2 g de borohydrure de sodium par fractions et en refroidissant. Après 2 heures on évapore le solvant sous pression réduite. On reprend le résidu dans 200 ml d'eau que l'on acidifie à pH 2 par de l'acide chlorhydrique, puis alcalinise au moyen d'une solution 12N d'hydroxyde de sodium. On extrait l'huile qui relargue avec 3 fois 200 ml d'acétate d'éthyle. On lave la phase organique à l'eau, la sèche sur du sulfate de magnésium et l'évapore à sec sous pression réduite. On recristallise le résidu dans de l'acétone. On obtient 6 g de produit que l'on transforme en chlorhydrate au sein de l'éthanol. Après recristallisation dans l'éthanol on obtient 2 g d'(isoquinolyl-1)-1-(pipéridyl-4)-3-propanol-1 sous forme de dichlorhydrate fondant à 210 °C.

L'(isoquinolyl-1)-1-(pipéridyl-4)-3-propanone-1
peut être préparée selon G.R.Clemo et coll. J. Chem. Soc. 1954, 95.

Exemple 7: (diméthoxy-6,7-isoquinolyl-1)-1-(pipéridyl-4)-3-propanol-1

A 5 g de (diméthoxy-6,7-isoquinolyl-1)-1-(pipéridyl-4)-3-propanone-1 dans 100 ml de méthanol on ajoute à la température ambiante 1,2 g de borohydrure de sodium.

Après 20 min on élimine le solvant sous pression réduite, reprend le résidu à l'eau et au chloroforme. On lave la phase organique à l'eau, la sèche sur du sulfate de magnésium et l'évapore à sec sous pression réduite. On obtient 5 g de produit que l'on redissout dans 50 ml d'éthanol, on ajoute de l'éthanol chlorhydrique jusqu'à pH 1 et 100 ml d'acétone. Après cristallisation, on filtre, lave les cristaux à l'acétone puis à l'éther.

Après séchage on obtient 4,4 g de (diméthoxy-6,7-isoquinolyl-1)-1-(pipéridyl-4)-3-propanol-1 sous forme de dichlorhydrate fondant à 192 °C.

Exemple 8: [(diméthyl-1,1-éthyl)-2-quinazolinyl-4]-1-(pipéridyl-4)-3-propanol-1

Dans 100 ml d'éthanol on introduit 2 g d'hydroxyde de sodium en pastilles, 7,6 g d'acétate de (diméthyl-1,1-éthyl)-2-quinazolinyl-4)-1-(pipéridyl-4)-3-propanone-1 et 0,8 g de borohydrure de sodium. Après 2 heures d'agitation on élimine l'éthanol sous pression réduite et reprend le résidu à l'eau et à l'acétate d'éthyle. On lave la phase organique à l'eau, la sèche sur du sulfate de magnésium et l'évapore à sec sous pression réduite.

On obtient 5,6 g de produit que l'on chromatographie sur gel de silice avec un mélange chloroforme-diéthylamine (97:3) comme éluant, on recueille 3,6 g de produit que l'on recristallise dans un mélange éther de pétrole-acétate d'éthyle. On obtient 1,6 g de [(diméthyl-1,1-éthyl)-2-quinazolinyl-4]-1-(pipéridyl-4)-3-propanol-1 fondant à 110°C.

Exemple 9: (naphthyl-2)-1-(pipéridyl-4)-3-propanamine-1

On porte 7 heures à 100° un mélange de 8,2 g de (naphthyl-2)-1-(pipéridyl-4)-3-propanone-1 et de 19 g de formiate d'ammonium. On reprend le résidu dans le chloroforme et le chromatographie sur du gel de silice avec un mélange toluène-éthanol-diéthylamine (15:2:1) comme éluant.

On récupère 7,2 g de produit que l'on traite par 70 ml d'acide chlorhydrique 6N et 20 ml d'acide acétique à l'ébullition pendant 22 heures. Après refroidissement on alcalinise le milieu réactionnel par une solution 12N d'hydroxyde de sodium et extrait l'huile qui relargue avec du chlorure de méthylène. On lave la phase organique à l'eau, la sèche sur du sulfate de magnésium et l'évapore à sec sous pression réduite. Par formation du dichlorhydrate au sein de l'isopropanol et recristallisation dans le n-propanol on obtient 2,5 g de (naphthyl-2)-1-(pipéridyl-4)-3-propanamine-1 sous forme de dichlorhydrate fondant à 260 °C.

Exemple 10: (isoquinolyl-1)-1-(pipéridyl-4)-3-propanamine-1

On porte 5 h à 160 °C un mélange de 2,5 g d'(isoquinolyl-1)-1-(pipéridyl-4-)3-propanone-1 et de 7,5 g de formiate d'ammonium. Après refroidissement on ajoute 100 ml d'eau et extrait d'insoluble par 3 fois 50 ml d'acétate d'éthyle. Après évaporation du solvant on reprend le résidu dans 35 ml d'acide chlorhydrique 6N et porte à ébullition 1 h. On dilue avec 100 ml d'eau, ajuste le pH de la solution aqueuse à 7 et extrait celle-ci au moyen d'acétate d'éthyle. On alcanise la phase aqueuse à pH 11 et l'extrait de nouveau avec 3 fois 100 ml de chloroforme. On sèche la phase organique sur du sulfate de magnésium et l'évapore à sec sous pression réduite. On obtient 0,13 g d'(isoquinolyl-1)-1-(pipéridyl-4)-3-propanamine-1 dont le spectre de R.M.N. du proton dans le chloroforme deutérié présente les caractéristiques suivantes:

$H_3$ δ: 8,5 ppm, $H_{4, 5, 6, 7}$ δ: entre 7,5 et 8 ppm. $H_8$ δ: 8,1 ppm

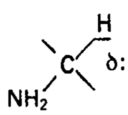 δ: 4,8 ppm,

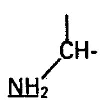 δ: 2,1 ppm

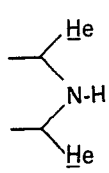 δ: 3 ppm

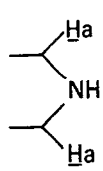 δ: 2,5 ppm

Propriétés pharmacologiques

Activité antiarythmique

L'activité antiarythmique des composés de formule (I) a été démontrée à l'aide du test à l'aconitine chez le rat.

Le principe de la technique repose sur le temps d'induction des arythmies ventriculaire provoquées par l'aconitine en perfusion lente chez le rat. Un substance antiarythmique retarde l'apparition des arythmies et ce délai est proportionnel à l'activité de la substance.

On utilise des groupes de 5 rats mâles. Une anesthésie individuelle est réalisée (uréthane 10%: 1 g/kg/ip) pour permettre une cathétérisation de la veine du pénis. L'électrocardiogramme est enregistré. Au temps T=0 la substance étudiée est injectée sous forme d'une solution aqueuse, à raison de 2,5 ml de solution par kg en 30 sec. Au temps T=60 sec, soit 30 sec après la fin de l'injection, l'aconitine est perfusée à raison de 20 μg par min, jusqu'à l'apparition d'extra systoles supraventriculaires. Le temps de perfusion de l'aconitine est noté.

On exprime les résultats par une $DE_{50}$, dose de produit en mg/kg qui, par rapport aux animaux témoins, augmente de 50% le temps de perfusion de l'aconitine.

Les résultats obtenus sont rassemblés dans le tableau ci-dessous:

| Produits | Test à l'aconitine (rat) $DE_{50}$ mg/kg i.v. |
|---|---|
| Exemple 1 | 4 |
| Exemple 2 | 3,8 |
| Exemple 3 | 2,1 |
| Exemple 5 | 1,42 |
| Exemple 6 | 2,7 |
| Exemple 7 | 2,5 |
| Exemple 8 | 1 |
| Exemple 9 | <0,3 |
| Quinidine | 7,5 |

Les composés de formule (I) présentent de remarquables propriétés antiarythmiques et sont plus actifs que la quinidine.

Propriétés toxocologiques

Les toxicités aiguës des composés de formule (I) ont été déterminées chez la souris mâle $CD_1$ (Charles River) par la voie intraveineuse. Les $DL_{50}$ calculées, après 3 jours d'observation, par la méthode cumulative de J.J. Reed et H. Muench (Amer. J. Hyg., 27, 493, 1938) sont supérieures à 15 mg/kg i.v.

Utilisation thérapeutique

Les composés de l'invention et leurs sels pharmaceutiquement acceptables peuvent être utilisés en thérapeutique humaine pour le traitement et/ou la prévention des troubles du rythme. Ils peuvent se présenter sous toutes les formes en usage dans le domaine des médicaments telles que comprimés, capsules, gélules, suppositoires, solutions ingérables ou injectables.

La posologie dépend des effects recherchés et de la voie d'administration utilisée. Par exemple, par voie orale, elle peut être comprise entre 50 et 800 mg de substance active par 24 heures, avec des doses unitaires variant de 10 à 100 mg de substance active.

Les exemples suivants illustrent des médicaments selon l'invention.

Exemple A: Soluté injectable

On prépare selon la technique habituelle une solution injectable contenant 10 mg de produit actif ayant la composition suivante:

| | |
|---|---|
| Composé de l'exemple 8 | 10 mg |
| Acide chlorhydrique N | 0,0375 ml |
| Acide ascorbique | 1 mg |
| Mannitol | 245 mg |
| Eau q.s. | 5 ml |

Exemple B: Comprimé

On prépare, selon la technique habituelle, des comprimés dosés à 25 mg de produit actif ayant la composition suivante:

| | |
|---|---|
| Composé de l'exemple 8 | 25 mg |
| Lactose | 52 mg |
| Cellulose | 20 mg |
| Polyvidone excipient | 5 mg |
| Carboxyme thylamidon sodique | 11 mg |
| Talc | 5 mg |
| Stéarate de magnésium | 1 mg |
| Silice colloidale | 1 mg |
| Hydroxypropyl methylcellulose-Glycérine et suspension d'oxide de titane q.s. | 123 mg |

Exemple C: Gélule

On prépare, selon la technique habituelle, des gélules dosées à 50 mg de produit actif ayant la composition suivante:

| | |
|---|---|
| Composé de l'exemple 8 | 50 mg |
| Lactose | 104 mg |
| Cellulose | 40 mg |
| Polyvidone excipient | 10 mg |
| Carboxyméthylamidon sodique | 22 mg |
| Talc | 10 mg |
| Stéarate de magnésium | 2 mg |
| Silice colloidale | 2 mg |

**Revendications pour les Etats contractants BE, CH, DE, FR, GB, IT, LI, LU, NL, SE**

1. Médicaments caractérisés en ce qu'ils contiennent comme substance active un composé de formule (I):

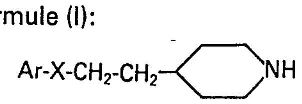
(I)

dans laquelle X représente un groupe -CO-, -CHOH- ou $-CH-NH_2$ et Ar représente un reste aromatique choisi parmi les cycles A, B, C ou D:

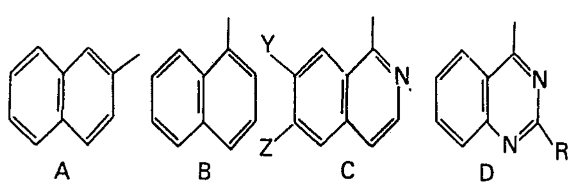
A B C D

dans lesquels R représente l'atome d'hydrogène ou un groupe alkyle comportant 1 à 4 atomes de carbone, Y et Z, identiques ou différents, représentent chacun l'atome d'hydrogène ou un groupe alcoxy comportant 1 à 3 atomes de carbone ou un sel d'un tel composé avec un acide pharmaceutiquement acceptable.

2. Composés de formule (I):

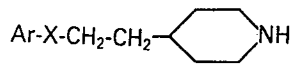
(I)

dans laquelle:

soit X représente le groupe -CHOH- ou $-CH-NH_2$ et Ar représente un reste aromatique choisi parmi les cycles A, B, C ou D selon la revendication 1 dans lesquels R représente l'atome d'hydrogène ou un groupe alkyle comportant 1 à 4 atomes de carbone, Y et Z, identiques ou différents, représentent chacun l'atome d'hydrogène ou un groupe alcoxy comportant 1 à 3 atomes de carbone,

soit X représente le groupe -CO- et Ar représente un reste aromatique choisi parmi les cycles A, C ou D mentionnés ci-dessus, excepte que Y et Z ne peuvent représenter tous les deux un atome d'hydrogène ou les sels de ces composés avec des acides minéraux ou organiques.

3. Procédé de préparation des composés selon la revendication 2 pour lesquels X est un groupe -CO- et Ar représente un reste aromatique choisi parmi les cycles A, C ou D excepté que Y et Z ne peuvent représenter tous les deux un atome d'hydrogène caractérisé en ce que l'on condense en présence d'une base, un ester de formule (II):

$$Ar\text{-}COOR_1 \qquad (II)$$

avec un ester d'acide (pipéridinyl-4)-propionique de formule (III):

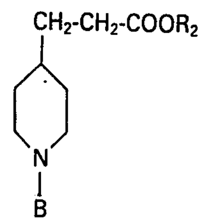
(III)

dans les formules (II) et (III), $R_1$ et $R_2$ représentent un groupe alkyle de bas poids moléculaire et Ar a la même signification que ci-dessus, B représente un groupe protecteur de la fonction amine stable en milieu alcalin anhydre; puis hydrolyse et décarboxyle le produit de condensation de formule (IV):

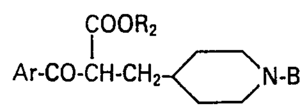
(IV)

obtenu précédemment au moyen d'une solution aqueuse d'un acide et transforme eventuellement en sel d'addition avec un acide.

4. Procédé de préparation des composés selon la revendication 2 pour lesquels X représente le groupe -CHOH- et Ar un reste aromatique choisi parmi les cycles A, B, C ou D caractérisé en ce que l'on réduit au moyen d'un hydrure métallique réducteur, les composés correspondants dans lesquels X est le groupe -CO- et transforme éventuellement en sel d'addition avec un acide.

5. Procédé de préparation des composés selon la revendication 2 pour lesquels X représente le groupe $-CH-NH_2$ et Ar un reste aromatique choisi parmi les cycles A, B, C et D caractérisé en ce que l'on traite les composés correspondants dans lesquels X est le groupe -CO- par du formiate d'ammonium puis hydrolyse en milieu acide et transforme éventuellement en sel d'addition avec un acide.

**Revendication pour l'Etat contractant AT**

1. Procédé de préparation de composés de formule (I):

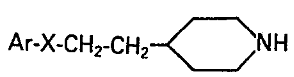

(I)

dans laquelle

a) soit X représente un groupe -CO- et Ar représente un reste aromatique choisi parmi les cycles A, C ou D:

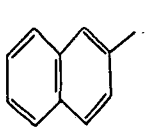

A

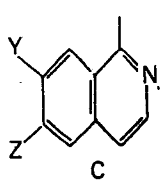

C

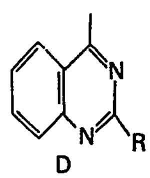

D

dans lesquels Y représente l'atome d'hydrogène ou un groupe alcoxy ayant 1 à 3 atomes de carbone, Z représente l'atome d'hydrogène ou un groupe alcoxy ayant 1 à 3 atomes de carbone excepté que Y et Z ne peuvent pas représenter tous les deux l'atome d'hydrogène et R représente l'atome d'hydrogène ou un groupe alkyle comportant 1 à 4 atomes de carbone,

b) soit X représente le groupe -CHOH- ou $-CH-NH_2$ et Ar représente un cycle aromatique choisi parmi les cycles A, B, C ou D:

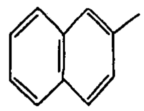

A

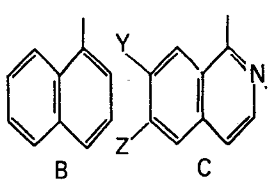

B C

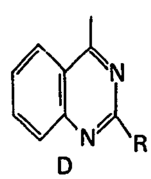

D

dans lesquels Y et Z, identiques ou différents, représentent l'atome d'hydrogène ou un groupe alcoxy ayant 1 à 3 atomes de carbone, R représente l'atome d'hydrogène ou un groupe alkyle comportant 1 à 4 atomes de carbone, ou les sels de ces composés avec des acides caractérisé en ce que:

A – Pour la préparation des composés de formule (I) définis précédemment en a), on condense en présence d'une base, un ester de formule (II):

$$Ar\text{-}COOR_1 \qquad (II)$$

dans laquelle Ar est défini comme précédemment en a) et $R_1$ représente un groupe alkyle comportant 1 ou 2 atomes de carbone avec un ester d'acide pipéridinyl-4 propionique de formule (III):

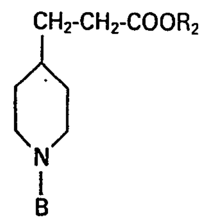

(III)

dans laquelle $R_2$ représente un groupe alkyle comportant 1 ou 2 atomes de carbone et B représente un groupe protecteur de la fonction amine stable en milieu alcalin anhydre, hydrolyse et décarboxyle le produit de condensation de formule (IV):

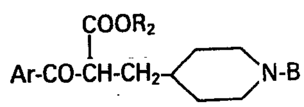

(IV)

ainsi obtenu au moyen d'une solution aqueuse d'un acide, et transforme éventuellement en sel d'addition avec un acide.

B – Pour la préparation des composés de formule générale (I) dans laquelle X représente le groupe -CHOH- et Ar est défini comme précédemment en b), on réduit au moyen d'un hydrure métallique réducteur les composés correspondants dans lesquels X est le groupe -CO- et transforme éventuellement en sel d'addition avec un acide.

C – Pour la préparation des composés de formule générale (I) dans laquelle X représente le groupe $-CH-NH_2$ et Ar est défini comme précédemment en b), on traite les composés correspondants dans lesquels X est le groupe -CO- par du formiate d'ammonium, puis hydrolyse et éventuellement transforme en sel d'addition avec un acide.

**Patentansprüche für die Vertragsstaaten BE, CH, DE, FR, GB, IT, LI, LU, NL, SE**

1. Arzneimittel, dadurch gekennzeichnet, dass sie als aktive Substanz eine Verbindung der Formel (I):

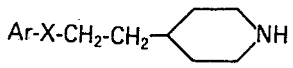 (I)

enthalten, worin X eine Gruppe -CO-, -CHOH- oder $-CH-NH_2$ bedeutet und Ar einen aromatischen Rest bedeutet, ausgewählt unter den Ringen A, B, C oder D:

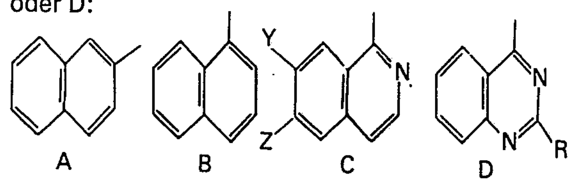

A B C D

worin R das Wasserstoffatom oder eine Alkylgruppe mit 1 bis 4 Kohlenstoffatomen bedeutet, Y und Z, die identisch oder verschieden sind, bedeuten jeweils das Wasserstoffatom oder eine Alkoxygruppe mit 1 bis 3 Kohlenstoffatomen oder ein Salz einer solchen Verbindung mit einer pharmazeutisch annehmbaren Säure.

2. Verbindungen der Formel (I):

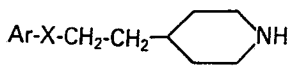 (I)

worin:

– entweder X die Gruppe -CHOH- oder $-CH-NH_2$ bedeutet und Ar einen aromatischen Rest bedeutet, ausgewählt unter den Ringen A, B, C oder D gemäss Anspruch 1, worin R das Wasserstoffatom oder eine Alkylgruppe mit 1 bis 4 Kohlenstoffatomen bedeutet, Y und Z, die identisch oder verschieden sind, jeweils das Wasserstoffatom oder eine Alkoxygruppe mit 1 bis 3 Kohlenstoffatomen bedeuten,

– oder X die Gruppe -CO- bedeutet und Ar einen aromatischen Rest, ausgewählt unter den oben erwähnten Ringen A, C oder D bedeutet, ausgenommen, dass Y und Z alle beide nicht ein Wasserstoffatom bedeuten können oder die Salze dieser Verbindungen mit mineralischen oder organischen Säuren.

3. Verfahren zur Herstellung der Verbindungen gemäss Anspruch 2, wobei X eine Gruppe -CO- ist und Ar einen aromatischen Rest, ausgewählt unter den Ringen A, C oder D, bedeutet, ausgenommen, dass Y und Z alle beide nicht ein Wasserstoffatom bedeuten können, dadurch gekennzeichnet, dass man in Gegenwart einer Base einen Ester der Formel (II):

$$Ar-COOR_1 \qquad (II)$$

mit einem Ester der (Piperidinyl-4)-propionsäure der Formel (III):

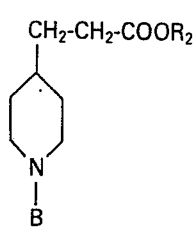 (III)

kondensiert, wobei in den Formeln (II) und (III) $R_1$ und $R_2$ eine Alkylgruppe mit niedrigem Molekulargewicht bedeuten und Ar dieselbe Bedeutung wie vorstehend hat, B eine Schutzgruppe der Aminfunktion, die in wasserfreiem alkalischem Milieu beständig ist, bedeutet; dass man dann das vorstehend erhaltene Kondensationsprodukt der Formel (IV)

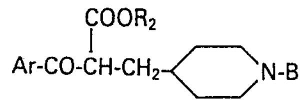 (IV)

mittels einer wässrigen Lösung einer Säure hydrolysiert und decarboxyliert und gegebenenfalls in ein Additionssalz mit einer Säure überführt.

4. Verfahren zur Herstellung der Verbindungen gemäss Anspruch 2, wobei X die Gruppe -CHOH- und Ar einen aromatischen Rest, ausgewählt unter den Ringen A, B, C oder D bedeutet, dadurch gekennzeichnet, dass man mittels eines reduzierenden Metallhydrids die entsprechenden Verbindungen, worin X die Gruppe -CO- ist, reduziert und gegebenenfalls in ein Additionssalz mit einer Säure überführt.

5. Verfahren zur Herstellung der Verbindungen gemäss Anspruch 2, wobei X die Gruppe $-CH-NH_2$ und Ar einen aromatischen Rest, ausgewählt unter den Ringen A, B, C und D, bedeutet, dadurch gekennzeichnet, dass man die entsprechenden Verbindungen, worin X die Gruppe -CO- ist, mit Ammoniumformiat behandelt, dann in saurem Milieu hydrolysiert und gegebenenfalls in ein Additionssalz mit einer Säure überführt.

**Patentanspruch für den Vertragsstaat AT**

Verfahren zur Herstellung von Verbindungen der Formel (I):

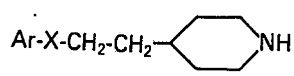 (I)

in welcher

a) entweder X eine Gruppe -CO- darstellt und Ar einen aromatischen Rest darstellt, ausgewählt aus den Ringen A, C oder D:

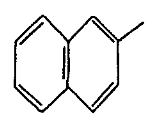
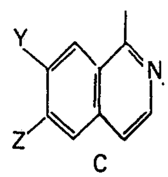
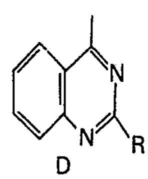

A C D

in welchen Y ein Wasserstoffatom oder eine Alkoxygruppe mit 1 bis 3 Kohlenstoffatomen darstellt, Z ein Wasserstoffatom oder eine Alkoxygruppe mit 1 bis 3 Kohlenstoffatomen darstellt, wobei Y und Z nicht gleichzeitig ein Wasserstoffatom darstellen dürfen, und R ein Wasserstoffatom oder eine Alkylgruppe mit 1 bis 4 Kohlenstoffatomen darstellt,

b) oder X die Gruppe -CHOH- oder $-CH-NH_2$ darstellt und Ar einen aromatischen Ring darstellt,

ausgewählt aus den Ringen A, B, C oder D:

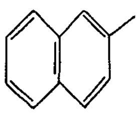 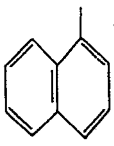 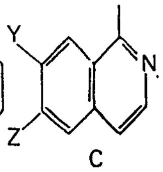 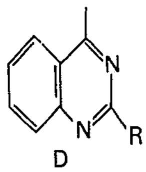

A B C D

in welchen Y und Z, die gleich oder voneinander verschieden sind, ein Wasserstoffatom oder eine Alkoxygruppe mit 1 bis 3 Kohlenstoffatomen darstellen und R ein Wasserstoffatom oder eine Alkylgruppe mit 1 bis 4 Kohlenstoffatomen darstellt, oder von den Salzen dieser Verbindungen mit Säuren, dadurch gekennzeichnet, dass man

A – zur Herstellung der zuvor unter a) definierten Verbindungen der Formel (I) einen Ester der Formel (II):

$Ar\text{-}COOR_1$ (II)

in welcher Ar die oben unter a) angegebene Bedeutung hat und $R_1$ eine Alkylgruppe mit 1 oder 2 Kohlenstoffatomen darstellt, in Gegenwart einer Base mit einem 4-Piperidinyl-propionsäureester der Formel (III)

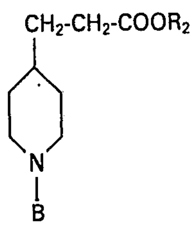 (III)

in welcher $R_2$ eine Alkylgruppe mit 1 oder 2 Kohlenstoffatomen darstellt und B eine in wasserfreiem, alkalischem Milieu stabile Schutzgruppe für die Aminfunktion darstellt, kondensiert, das so erhaltene Kondensationsprodukt der Formel (IV):

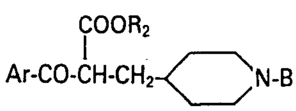 (IV)

mittels einer wässrigen Lösung einer Säure hydrolysiert und decarboxyliert und gegebenenfalls in ein Säureadditionssalz überführt,

B – zur Herstellung von Verbindungen der allgemeinen Formel (I), in welcher X die Gruppe -CHOH- darstellt und Ar die oben unter b) angegebene Bedeutung hat, die entsprechenden Verbindungen, in welchen X die Gruppe -CO- ist, mittels eines Metallhydrid-Reduktionsmittels reduziert und gegebenenfalls in ein Säureadditionssalz überführt,

C – zur Herstellung von Verbindungen der allgemeinen Formel (I), in welcher X die Gruppe $-CH\text{-}NH_2$ darstellt und Ar die oben unter b) angegebene Bedeutung hat, die entsprechenden Verbindungen, in welchen X die Gruppe -CO- ist, mit Ammoniumformiat behandelt und dann hydrolysiert und gegebenenfalls in ein Säureadditionssalz überführt.

**Claims for the contracting States BE, CH, DE, FR, GB, IT, LI, LU, NL, SE**

1. A drug which contains as active substance a compound of formula (I):

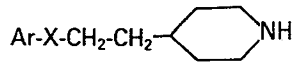 (I)

in which X denotes a -CO-, -CHOH- or $-CH\text{-}NH_2$ group and Ar denotes an aromatic residue chosen from the ring systems A, B, C or D:

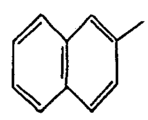 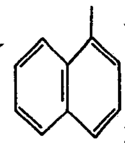 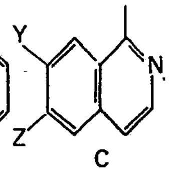 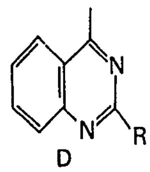

A B C D

in which R denotes a hydrogen atom or an alkyl group containing 1 to 4 carbon atoms, and Y and Z, which may be identical or different, each denote a hydrogen atom or an alkoxy group containing 1 to 3 carbon atoms, or a salt of such a compound with a pharmaceutically acceptable acid.

2. A compound of the formula (I):

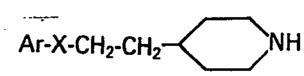

in which:

either X denotes a -CHOH- or $-CH\text{-}NH_2$ group and Ar denotes an aromatic residue chosen from the ring systems A, B, C or D according to claim 1 in which R denotes a hydrogen atom or an alkyl group containing 1 to 4 carbon atoms, and Y and Z, which may be identical or different, each denote a hydrogen atom or an alkoxy group containing 1 to 3 carbon atoms,

or X denotes a -CO- group and Ar denotes an aromatic residue chosen, from the ring systems A, C or D mentioned above, except that Y and Z cannot both denote a hydrogen atom, or the salts of these compounds with inorganic or organic acids.

3. A process for preparing the compound according to claim 2 for which X is a -CO- group and Ar denotes an aromatic residue chosen from the ring systems A, C or D except that Y and Z cannot both denote a hydrogen atom, wherein an ester of formula (II):

$Ar\text{-}COOR_1$ (II)

is condensed, in the presence of a base, with an ester of (4-piperidyl)propionic acid of formula (III):

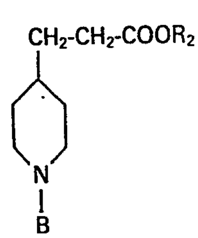 (III)

in the formula (II) and (III) $R_1$ and $R_2$ denote a low molecular weight alkyl group and Ar has the same meaning as above, B denotes a group which protects the amine group and which is stable in anhydrous alkaline medium; the condensation product of formula (IV):

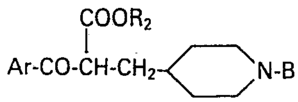 (IV)

obtained above is then hydrolysed and decarboxylated using an aqueous solution of an acid and, where appropriate, converted to an addition salt with an acid.

4. A process for preparing the compound according to claim 2 for which X denotes a -CHOH- group and Ar an aromatic residue chosen from the ring systems A, B, C or D, wherein the corresponding compound in which X is a -CO- group is reduced using a reducing metal hydride and, where appropriate, converted to an addition salt with an acid.

5. A process for preparing the compound according to claim 2 for which X denotes a -CH-$NH_2$ group and Ar an aromatic residue chosen from the ring systems A, B, C and D, wherein the corresponding compound in which X is a -CO- group is treated with ammonium formate and then hydrolysed in acid medium and, where appropriate, converted to an addition salt with an acid.

**Claim for the contracting State AT**

A process for preparing a compound of formula (I):

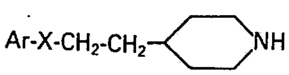 (I)

in which

a) either X denotes a -CO- group and Ar denotes an aromatic residue chosen from the ring systems A, C, or D:

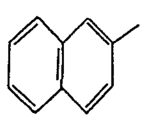
A

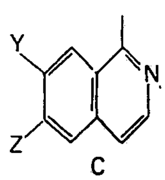
C

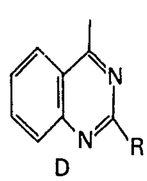
D

in which Y denotes a hydrogen atom or an alkoxy group having 1 to 3 carbon atoms, Z denotes a hydrogen atom or an alkoxy group having 1 to 3 carbon atoms, except that Y and Z cannot both denote a hydrogen atom, and R denotes a hydrogen atom or an alkyl group containing 1 to 4 carbon atoms,

b) or X denotes a -CHOH- or -CH-$NH_2$ group and Ar denotes an aromatic ring system chosen from the ring systems A, B, C or D:

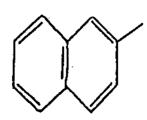
A

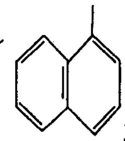
B

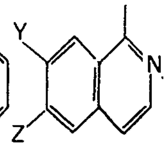
C

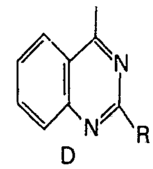
D

in which Y and Z, which may be identical or different, denote a hydrogen atom or an alkoxy group having 1 to 3 carbon atoms, R denotes a hydrogen atom or an alkyl group containing 1 to 4 carbon atoms, or the salts of these compounds with acids, wherein:

A – For preparing the compound of formula (I) defined above in a), an ester of formula (II):

$$Ar\text{-}COOR_1 \qquad (II)$$

in which Ar is as defined above in a) and $R_1$ denotes an alkyl group containing 1 or 2 carbon atoms is condensed, in the presence of a base, with an ester of 4-piperidylpropionic acid of formula (III):

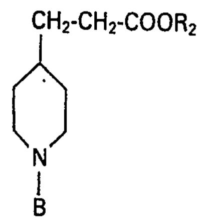 (III)

in which $R_2$ denotes an alkyl group containing 1 or 2 carbon atoms and B denotes a group which protects the amine group and which is stable an anhydrous alkaline medium, the condensation product of formula (IV):

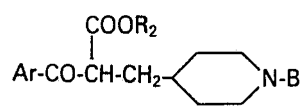 (IV)

obtained in this way is hydrolysed and decarboxylated using an aqueous solution of an acid, and, where appropriate, converted to an addition salt with an acid.

B – For preparing the compound of general formula (I) in which X denotes a -CHOH- group and Ar is as defined above in b), the corresponding compound in which X is a -CO- group is reduced using a reducing metal hydride and, where appropriate, converted to an addition salt with an acid.

C – For preparing the compound of general formula (I) in which X denotes a -CH-$NH_2$ group and Ar is as defined above in b), the corresponding compound in which X is a -CO- group is treated with ammonium formate, and then hydrolysed and, where appropriate, converted to an addition salt with an acid.

PLANCHE 1/I

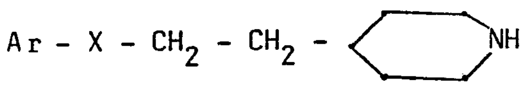

(I)

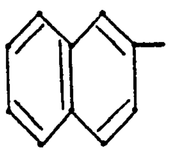
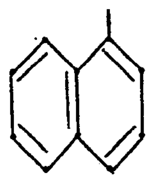
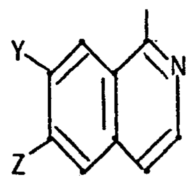
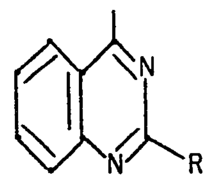

A B C D

$Ar - COOR_1$

(II)

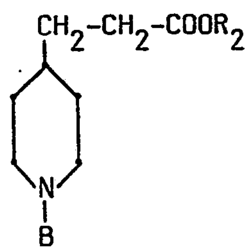

(III)

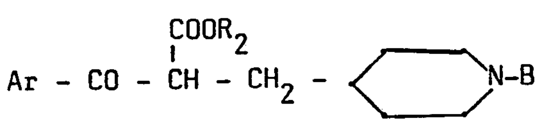

(IV)